# EUROPEAN PATENT APPLICATION

(11) **EP 1 512 381 A2**
(43) Date of publication of application: **09.03.2005**
(21) Application number: 04255234.9
(22) Date of filing: 28.08.2004
(51) Int. Cl.: A61F 2/06

(54) **Side branch stent with split proximal end**

(30) Priority: 03.09.2003 US 654118
(71) Applicant: Cordis Corporation, Miami Lakes Florida 33014 (US)
(72) Inventor: Fischell, Tim A., Richland, Michigan 49083 (US); Fischell, David R., Fair Haven, New Jersey 07704 (US); Burgermeister, Robert, Bridgewater, New Jersey 08807 (US); Grishaber, Randy-David Burce, Asbury, New Jersey 08802 (US)
(74) Representative: Belcher, Simon James

(57) **Abstract**

Two stents for implantation into a vessel of a human body are described. The first stent is in the form of a longitudinal axis, a proximal end and a distal end. The stent has a distal section comprising a plurality of circumferential sets (12) of strut members. Each set of strut members is longitudinally separated each from the other and each set of strut members forms a cylindrical portion of the stent. The stent also has a proximal section comprising at least three spokes (15). Each spoke is connected to the proximal-most circumferential set (18) of strut members of the distal section of the stent.

The second stent also has a longitudinal axis, a proximal end and a distal end. The stent has a cylindrical distal section and a split proximal section designed to be flared outward with respect to the cylindrical distal section.

## Description

It has been shown that intravascular stents are an excellent means to maintain the patency of blood vessels following balloon angioplasty. As stent technology has advanced, more and more complex anatomy has been treatable with stents.

A particularly difficult anatomy to treat is that of a bifurcation in a blood vessel at the ostium of a side branch. Fischell et al., U.S. Pat. No. 5,749,825, describe a stent system for bifurcations. The Fischell design has two guide wire lumens allowing the deployment of a stent in the first blood vessel while leaving a guide wire positioned through the stent struts into the second vessel which is a side branch. In Fischell the profile (outside diameter) of the stenting system is significantly larger as compared to a stent delivery catheter that uses a single guide wire. In addition, Fischell does not address placement of a stent into the second branch (across the ostium,) which is often not at a 90-degree angle to the first vessel.

A bifurcation stent delivery catheter with two distal balloons and one stent segment for each of the two vessels would address the issue of stenting the second branch vessel but such a device would be quite large in profile and extremely hard to deliver. If one places a first stent into a main artery with that stent being positioned across the ostium of the side branch and the side branch is not at a 90-degree angle to the main branch, then either the second stent will extend into the main branch of the artery, or some portion of the arterial wall at the ostium will not be properly supported by the second stent.

Published US Patent Application No. US-2003/0050688-A1 describes a stent with an angulated proximal end to better align with the angled opening of a sidebranch vessel. Unfortunately, positioning a balloon expandable stent of this type is not easy. Even if the stent delivery system can be rotated to properly orient the proximal end of the stent, there is often a rotational effect on the stent during balloon unfolding during stent deployment. Others have developed a "stent-crush" technique, to be used with drug eluting stents. In this technique, a first stent is implanted into the sidebranch with its proximal end extending out into the main branch. A second stent is then implanted in the main branch, crushing flat the proximal end of the sidebranch stent. A guidewire is the advanced through the side of the main branch stent and the flattened area of the crushed sidebranch stent into the sidebranch. A balloon expansion of the "crushed" area, "unjails" (opens) the stent for blood flow. However, this method leaves three layers of stent metal at the proximal end of the sidebranch stent, [i.e., both sides of the crushed sidebranch stent and the single layer of the main branch stent.] This is particularly troublesome with cytotoxic drug eluting stents (e.g. paclitaxel) where the proper dosing occurs over a narrow range.

Most current tubular stents use a multiplicity of circumferential sets of strut members connected by either straight longitudinal connecting links or undulating longitudinal flexible links. The circumferential sets of strut members are typically formed from a series of diagonal sections connected to curved sections forming a circumferential, closed-ring, zig-zag structure. This structure expands as the stent deploys, to form the elements of the stent that provide structural support for the arterial wall.

The terms "side branch" and "bifurcation" will be used interchangeably throughout this specification.

It is highly desirable after placing a first stent into the "main branch" of an artery and inserting a guide wire through the side of the expanded stent and into a side branch, to be able to place a stent across the ostium of the angled side branch (or bifurcation) where the second stent provides support to scaffold the arterial wall at the ostium of the side branch without having the stent extend into the main branch. The present invention uses a stent with a proximal end designed to be "split" apart, to provide metal coverage and drug delivery to the entire ostium of the sidebranch while avoiding the triple metal issue of using the crush technique. Such a stent could be self-expanding with its proximal end designed to flare apart when released from the stent delivery sheath, or it may be balloon expandable.

The self-expanding embodiment would typically be made from a nickel titanium shape memory alloy, such as nitinol having a transition temperature above body temperature. The delivery catheter for this embodiment would typically have 3 radiopaque markers designed to indicate the distal end of the stent, the proximal end of the stent and the distal end of the proximal stent section designed to split apart upon deployment. In another embodiment of the self-expanding version of the present invention stent, just the proximal, split end section would have a transition temperature slightly above body temperature. After delivery of the stent into the sidebranch, there would be an added step of heating the most proximal section of the self-expanding above its transition temperature, to cause this section to flare. This could be accomplished by injecting saline solution at above the transition temperature of the proximal section of the stent.

The balloon expandable embodiment can be delivered on a standard balloon stent delivery system. Or, it is envisioned that a stent delivery system may have at least two balloon sections, with the proximal section having a greater diameter than the distal section, so as to spread apart the split proximal end of the present invention stent. The balloon proximal section might also be much more compliant than the distal section, so that as higher pressures are used, the ratio of the diameter of the proximal balloon section to the distal balloon section increases. The stent delivery system would also have three marker bands, to allow the user to visualize the distal end of the stent, the proximal end of the stent and the distal end of the "split" proximal end.

The method for use of the present invention stent and stent delivery system is to insert a first guidewire into the branch vessel and advance the stent delivery system until the marker at the distal end of the split proximal end is aligned with the downstream edge of the sidebranch ostium. The balloon is then inflated to deliver the stent into the sidebranch. If the two zone balloon is being used as a stent delivery system, the initial inflation will cause the split proximal end to flare apart. If the stent is delivered on a standard balloon angioplasty catheter, then a second balloon of larger diameter would be used to post-dilate the proximal end of the split end stent. A stent is then advanced into the main branch and deployed, further spreading the split proximal end of the split end sidebranch stent outward against the wall of the main branch. A guidewire is then placed through the main branch stent and the opening into the sidebranch is enlarged using balloon inflation. The final result is a double layer of metal at the ostium of the sidebranch where additional anti-restenosis drug elution is desirable.

As with most current stents, the present invention stent uses longitudinally connected circumferential sets of strut members formed from alternating curved sections (crowns) and straight sections. It is the circumferential sets of strut members that form the support structure of the stent.

In one embodiment of the current invention the proximal split end section of the stent has the same number of spokes as crowns (curved sections) in the most proximal circumferential set of strut members. In an alternate embodiment there is one spoke for every two crowns in the most proximal circumferential set of strut members. It is also envisioned that more than one spoke per crown is possible, although a ratio of one-half or one will best fit typical coronary stent geometry. For example in a typical closed cell 3mm diameter coronary stent such as that sold under the mark CORDIS BX Velocity stent (Miami Lakes, Florida), there are 6 cells (i.e. 12 crowns) in each circumferential set of strut members. Thus either 6 or 12 spokes would typically be used. The 12-spoke embodiment will provide a better spread of coverage, while the 6-spoke is easier to manufacture. For smaller diameter vessels, as few as 4 spokes might be used, and for larger vessels as many as 18 spokes might be used.

In another embodiment of the present invention, the split ends are connected to an extra long circumferential set of strut members that will further connect the ends of the individual spokes of the split end stent and would enhance stent retention at the stent proximal end as compared with unconnected spokes.

Thus it is an object of this invention to have a split end sidebranch stent having a split proximal section designed to act as a set of spreadable spokes.

Another object of this invention is to have a stent delivery system with a larger diameter proximal section designed to spread the spokes of the split proximal end of a split end sidebranch stent.

Still another object of the present invention is to have a self-expanding split end stent with the proximal split end section "pre-set" to flare outward when released.

Yet another object of the present invention is to have a self-expanding split end stent formed from a nickel titanium based shape memory alloy, such as nitinol, with the proximal split end section having a transition temperature above body temperature.

Another object of this invention is to have the proximal-most circumferential set of strut members connected to each of the spreadable spokes, the circumferential set of strut members being of much greater length than all other circumferential sets of strut members in the stent.

A final object of this invention is to have a method for deploying a stents at a bifurcation or sidebranch where a first split end sidebranch stent is delivered into the sidebranch, the proximal spreadable spokes are flared out, and then a second stent is delivered into the main branch, the second stent pressing the flared spokes into the arterial wall. The sidebranch is then "unjailed" in the standard manner, and the procedure is complete.

These and other objects and advantages of this invention will become apparent upon reading of the detailed description of this invention, including the associated drawings.

Embodiments of the invention will now be described by way of example with reference to the accompanying drawings, in which:
FIG. 1A is cross section of an artery with a side branch where a first stent is placed into the main branch and a second stent is placed into a side branch leaving part of the wall of the side branch unsupported;
FIG. 1B is a cross section of an artery with a side branch where a first stent is placed in the main branch and a second stent is placed into a side branch where a proximal portion of the second stent extends part way into the main branch;
FIGs 2A, 2B and 2C show the "crush" technique for sidebranch stenting where FIG. 2A shows a first stent delivered into a sidebranch;
FIG. 2B shows a second stent delivered into the main branch crushing flat the proximal end of the first stent;
FIG 2C shows a guidewire in place to unjail the sidebranch;
FIG. 3 is a flat layout view of one embodiment of the present invention split end stent;
FIG. 4 is a three dimensional sketch of the present invention split end stent of FIG. 3 after it has been cut and electropolished but before it is mounted on a stent delivery catheter;
FIG. 5A shows the use of a second balloon catheter to spread the spokes of the proximal section of the balloon expandable split end stent following its delivery into a side branch vessel;
FIG. 5B shows the split end stent with flared proximal spokes just before stenting of the main branch vessel;
FIG. 5C shows the deployment of a balloon expandable stent into the main branch vessel;
FIG. 5D shows the resulting two stent structure with a guidewire placed through a cell of the main branch stent into the side branch just before final unj ailing of the side branch by balloon expansion of the cell in the main branch stent;
FIG. 6 is a flat layout of another embodiment of the present invention split end stent having two spokes for each crown in the most proximal circumferential set of strut members;
FIG. 7 is a flat layout of another embodiment of the split end stent having a proximal end circumferential set of strut members designed to accommodate the flaring of the proximal end;
FIG. 8 is a close-up of area "8" of FIG. 7, showing a tapered connection point designed to provide strain relief between the proximal circumferential set of strut members and the spokes;
FIG. 9 is a longitudinal cross section of the post deployment configuration of the present invention balloon expandable split end stent with stent delivery system where the stent delivery system has a larger diameter proximal section designed to flare the proximal spokes;

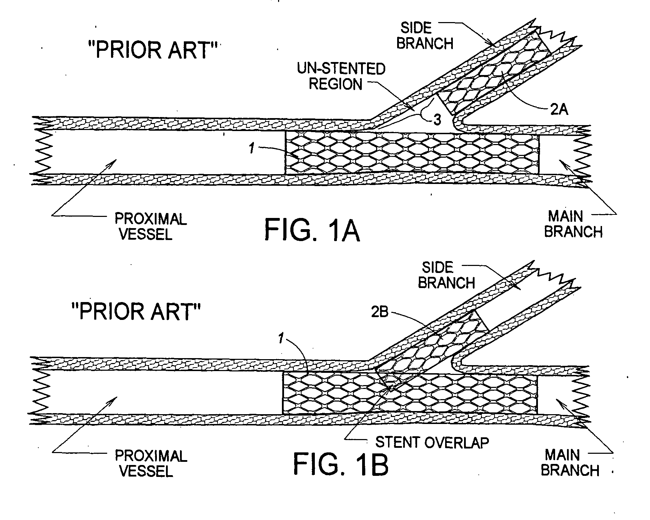

FIG. 1A shows an artery with a side branch, (i.e., a bifurcated artery) where a first "prior art" stent 1 has been deployed into the main branch and a second "prior art" stent 2A has been deployed into the side branch leaving the section 3 of the arterial wall at the ostium of the side branch un-stented and therefore unsupported. The side branch vessel attaches to the main branch at an acute angle f that is less than 90 degrees. An angle f=90 degrees would be a perpendicular attachment.

FIG. 1B shows an artery with side branch where a first "prior art" stent 1 has been deployed into the main branch and a second "prior art" stent 2B has been deployed into the side branch. In FIG. 1B, the second stent 2B extends part way into the main branch causing the stents to overlap. Such an extension of metal into an artery can cause turbulent blood flow that can readily result in subacute thrombosis.

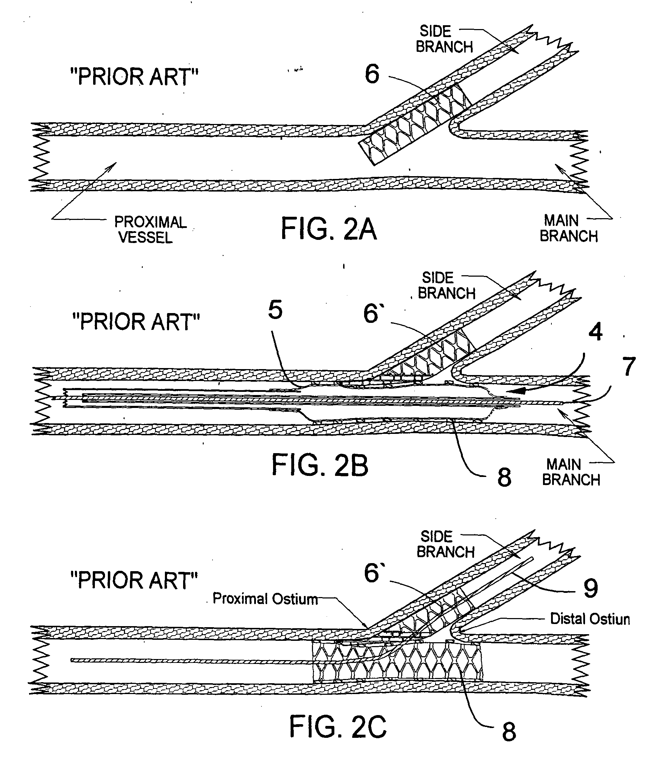

FIGS. 2A through 2C are an illustration of the "crush" method for stenting a sidebranch vessel. In the first step shown in FIG. 2A, a stent 6 is expanded into the ostium of the side branch with a significant portion of the stent 6 protruding into the main branch. Next as shown in FIG. 2B, a second stent 8 is expanded by a stent delivery catheter 4 with balloon 5 into the main branch. This expansion produces the crushed stent 6' having a flattened proximal end. Finally as shown in FIG. 2C, a guidewire 9 is placed through a cell in the side of the stent 8, through the "crushed" stent 6' and into the side branch vessel. This will allow a final step (not shown) of balloon expansion to "unjail" the ostium of the side branch. This technique, while it does overcome the situation shown in FIGS. 1A and 1B leaves three layers of metal at the proximal junction of the main branch and side branch. If drug eluting stents are used to reduce restenosis in the stented side branch, there would be a potentially very high dose area created by the triple layers of metal.

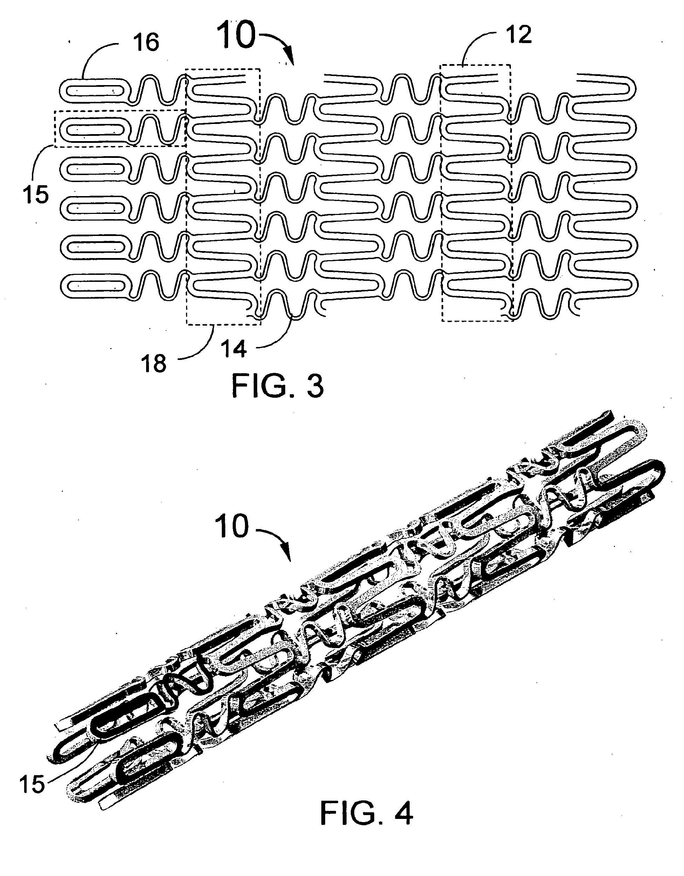

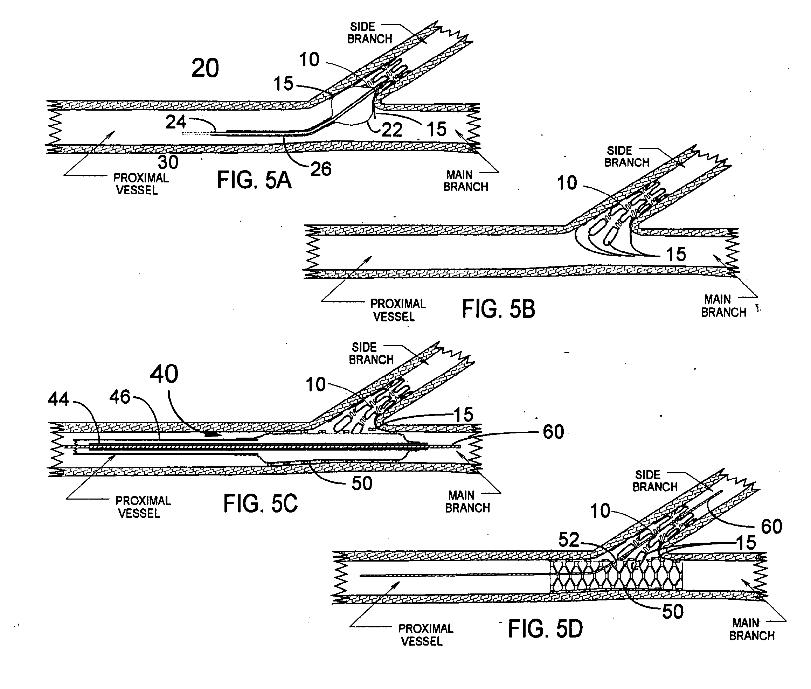

FIG. 3 is a flat layout view of one embodiment of the present invention split end stent 10. The distal section of the stent is similar to that of the stent sold under the mark CORDIS BX Velocity, having a multiplicity of circumferential sets of strut members 12 connected by undulating flexible links 14. The proximal end of the stent 10 has six spokes 15. Each spoke 15 is composed of an end segment 16 and an undulating flexible link 14 that connects the end segments 16 to the most proximal circumferential set of strut members 18. The spokes 15 are designed to be spread outward by a balloon during the stenting procedure (see FIGS. 5A through 5D) so as to effectively cover the ostium of a side branch. The end segments 16 have a shape designed to provide sufficient surface area for an effective dose from a drug eluting coating where the drug may be a cytostatic drug such as sirolimus, everolimus, the expanded drug ABT-578 or other sirolimus analogues, or it may be a cytotoxic drug such as paclitaxel. It is also envisioned that an outer heparin surface coating may be applied to reduce the potential for sub-acute thrombosis.

FIG. 4 is a three dimensional sketch of the present invention split end stent 10 of FIG. 3 with spokes 15 at the proximal end of the stent. FIG. 4 shows the configuration of the stent 10 after it has been cut and electropolished, but before it is mounted on a stent delivery catheter.

FIGs. 5A through 5D show steps in the stenting of a sidebranch/bifurcation using the stent 10 of FIGs. 3 and 4. Before FIG. 5A, the split end stent 10 of FIGs. 2 and 3 is deployed at the ostium of main branch and sidebranch vessels.

FIG. 5A shows the use of a balloon catheter 20 to spread the spokes 15 of the proximal section of the balloon expandable split end stent 10 following an earlier deployment of the stent 10 into the ostium of the side branch vessel. The balloon catheter 20 has balloon 22 inner shaft 24 and outer shaft 26. The balloon catheter 20 is inserted into the ostium of the sidebranch over the guide wire 30.

FIG. 5B shows the split end stent 10 with flared proximal spokes 15 after removal of the balloon catheter 20 and just before stenting of the main branch vessel.

FIG. 5C shows the deployment of a balloon expandable stent 50 into the main branch vessel with the stent delivery system 40 having balloon 42, inner shaft 44 and outer shaft 46. The stent delivery system 40 is advanced to the deployment site over the guidewire 60.

FIG. 5D shows the resulting two stent structure with a guidewire placed through a cell 50 of the main branch stent 50 through the side branch stent 10 and into the side branch just before final unjailing of the side branch by balloon expansion of the cell 52 in the main branch stent 50.

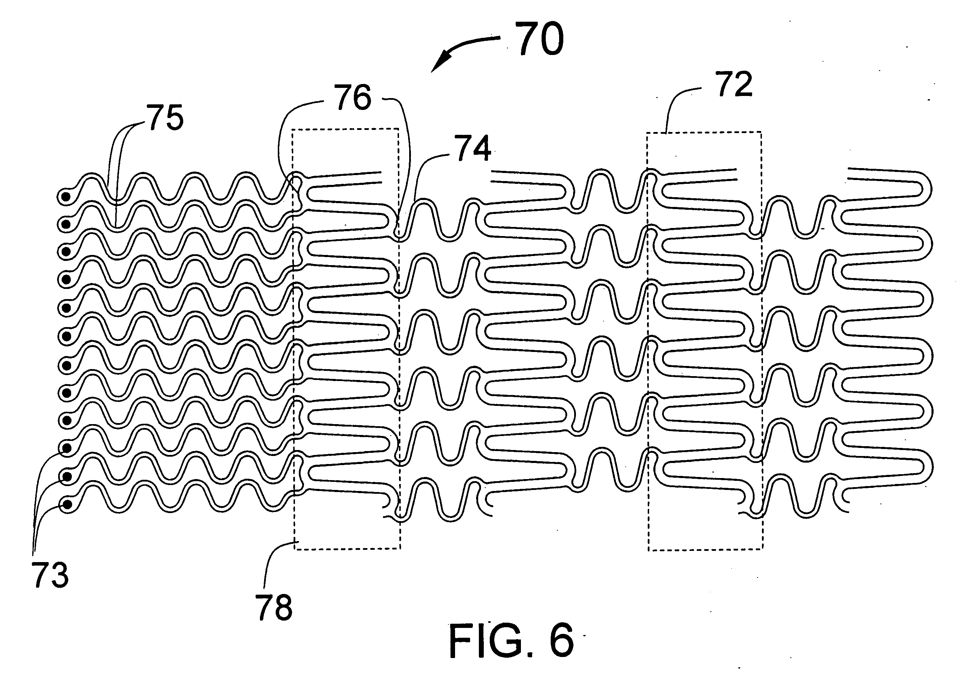

FIG. 6 is a flat layout of another embodiment of the present invention split end stent 70 having one spoke 75 for each crown 76 in the most proximal circumferential set of strut members 78. The widened proximal end of each spoke 75 has a radiopaque insert 73, such as is used in the stent sold under the mark CORDIS SMARTER. The radiopaque insert 73 would facilitate visualization of the flared spokes to ensure proper deployment. Examples of typical materials used in the radiopaque insert would be a radiopaque metal such as gold, platinum or tantalum, or alloys such as the carat-gold and platinum-iridium alloys. Further, elemental Palladium and Iridium are biocompatible materials that would exhibit desirable radiopacity, as does tungsten, which could be sputtered onto the stent surface.

Although every spoke 75 of the stent 70 has a radiopaque marker 73, it is also envisioned that only a subset of the spokes, (e.g. half of the spokes) might have the radiopaque inserts. It is also envisioned that radiopaque inserts might be added to any of the embodiments of the stents described herein.

Although each spoke 75 has an undulating shape to allow it the flexibility to adapt to the shape of the side branch ostium, it is envisioned that straight spokes could also work, especially if the stent has a relatively thin wall (e.g. less than 0.1 mm (0.004")). The stent 70 with two spokes 75 for each crown 76 would provide a better metal coverage and therefore more uniform drug delivery at the ostium of a side branch than the stent 10 as the stent 70 has twice the number of spokes as the stent 10.

As with earlier embodiments, the stent 70 has a distal section constructed of circumferential sets of strut members 72, connected by a multiplicity of undulating flexible links 75. Although each of the embodiments shown herein has a distal section that is a closed cell design, a split proximal end type design is equally applied to open cell stents.

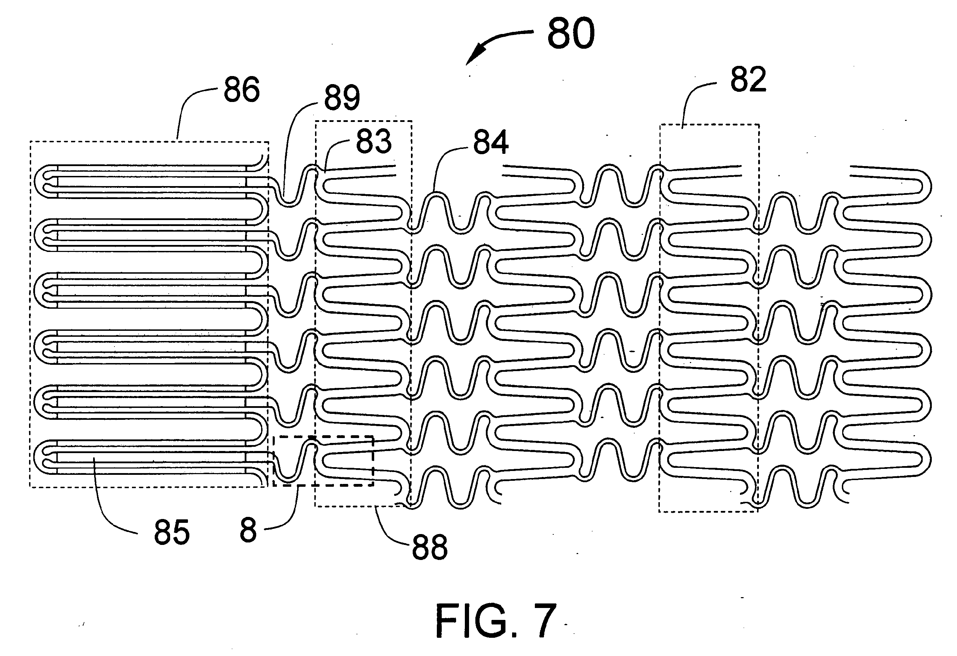

FIG. 7 is a flat layout of still another embodiment of the split end stent 80 having a proximal end circumferential set of strut members 86 having sufficient length to accommodate the flaring of the proximal end. The proximal section of the stent 80 is similar to that of the stents 10 and 70 of FIGS. 3 and 6 respectively having circumferential sets of strut members 82 connected by a multiplicity of undulating flexible links 84. The flare-able proximal end circumferential set of strut members 86 attaches to the most proximal standard circumferential set of strut members 88 via flexible links 89. For better visualization of the flared proximal end of the stent 80, the mid portions of the proximal circumferential set of strut members 86 have been coated with a radiopaque material 85 such as gold or platinum. It should be noted that only the straight sections of the circumferential set of strut members 86 have been coated to avoid any potential cracking of the coating during expansion of the stent 80. It is also envisioned that such a radiopaque marking could be applied to the proximal spokes of any of the other embodiments described herein such as the stent 10 of FIG. 3 and/or the stent 70 of FIG. 6.

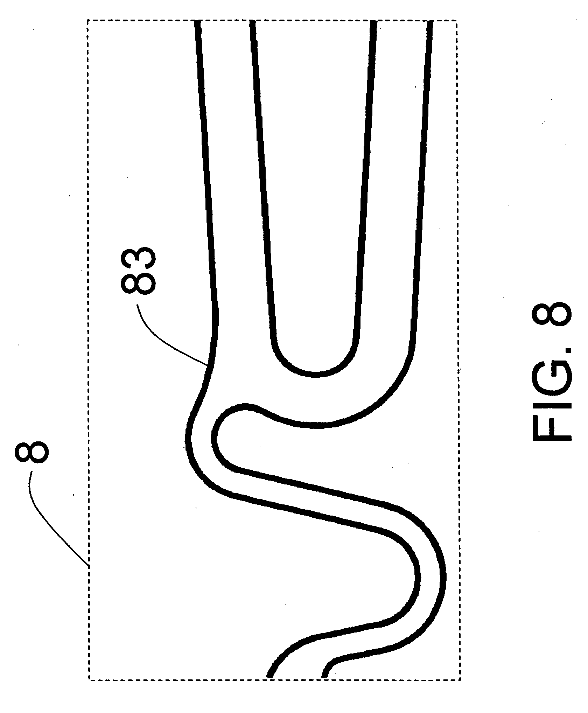

The connection 83 of the links 89 to the most proximal circumferential set of strut members 88 of the stent 80 has a tapered shape to provide strain relief. This tapered shape is seen more clearly in the close up of the section 8 shown in FIG. 8.

Although the flexible links 89 of the stent 80 have an undulating shape, straight connecting links would also work with this design. The advantage of the design of the stent 80 over the embodiments illustrated by the stents 10 and 70 is that in having a closed circumferential structure at its proximal end, the stent 80 will be better retained when crimped on a balloon stent delivery catheter.

The stents 10, 70 and 80 can be either balloon expandable or self-expanding. If balloon expandable, the stents should be made from a biocompatible metal such as stainless steel, tantalum or cobalt-chromium alloys. If self-expanding, the stents would typically be made from a nickel titanium based shape memory alloy, such as nitinol.

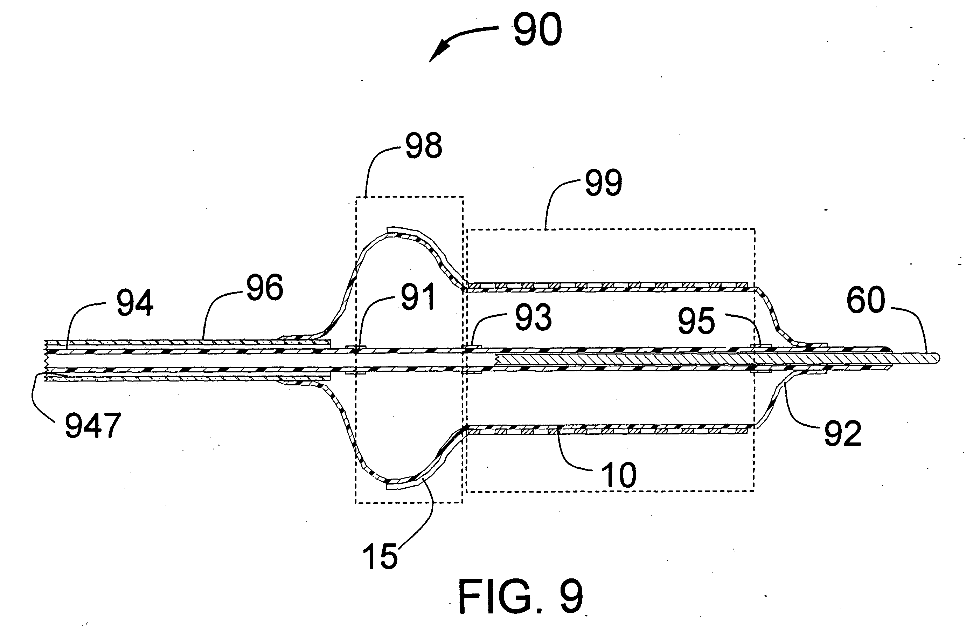

FIG. 9 is a longitudinal cross section of the post deployment configuration of the present invention balloon expandable split end stent 10 with stent delivery system 90 where the stent delivery system 90 has a balloon 92 with a cylindrical shaped distal section 99 and larger diameter proximal section 98, designed to flare the proximal spokes 15 of the stent 10. The stent delivery system 90 is designed to be advanced over a guidewire 60, and has a distal end construction similar to that of standard balloon angioplasty catheters. A balloon inflation lumen 97 is placed between the inner shaft 94 and outer shaft 96. The balloon 92 attaches at its proximal end to the outer shaft 96 and at its distal end to the inner shaft 94.

Unlike standard balloon stent delivery systems that have two radiopaque marker bands, the stent delivery system 90 utilizes three radiopaque marker bands 91, 93 and 95. The marker band 91 marks the proximal end of the mounted stent 10 and the marker band 95 marks the distal end of the mounted stent 10. The marker band 93 marks the distal end of the spokes 15, and can be used to properly position the stent delivery system 90 before stent deployment at the ostium of a side branch vessel. The stent delivery system 90 has a proximal section (not shown) that can be in the form of a standard over-the-wire stent delivery system or a standard rapid exchange stent delivery system.

Although the stent delivery system 90 is ideally suited to delivery a split end stent such as the stents 10, 70 or 80, it is envisioned that the stents might be delivered on a standard stent delivery system without the flaring section 98 of the stent delivery system 90. A second balloon would then be required to flare the split proximal end as shown in FIG. 5A.

## Claims

1. A stent for implantation into a vessel of a human body, the stent having a longitudinal axis, a proximal end and a distal end, the stent having a distal section comprising a plurality of circumferential sets of strut members, each set of strut members being longitudinally separated each from the other and each set of strut members forming a cylindrical portion of the stent, the stent also having a proximal section comprising at least three spokes, each spoke connected to the proximal-most circumferential set of strut members of the distal section of the stent.

2. The stent of claim 1 wherein the stent is self-expanding.

3. The stent of claim 1 wherein the stent is balloon expandable.

4. The stent of claim 1 wherein each spoke is only attached to the proximal-most circumferential set of strut members of the distal section of the stent.

5. The stent of claim 1 wherein each spoke is connected to adjacent spokes by strut members, the strut members collectively forming a circumferential set of strut members at the proximal end of the stent.

6. The stent of claim 1 where each circumferential set of strut members comprises a plurality of connected curved sections, and the number of spokes is equal to the number of curved sections in the proximal-most circumferential set of strut members of the distal section of the stent.

7. The stent of claim 1 where each circumferential set of strut members comprises a multiplicity of connected curved sections and the number of spokes is less than the number of curved sections in the proximal-most circumferential set of strut members of the distal section of the stent.

8. The stent of claim 1 where each circumferential set of strut members comprises a multiplicity of connected curved sections and the number of spokes is more than the number of curved sections in the proximal-most circumferential set of strut members of the distal section of the stent.

9. The stent of claim 1 further comprising a stent delivery system having three radiopaque markers for positioning the stent at the ostium of a side branch vessel.

10. A stent for implantation into a vessel of a human body, the stent having a longitudinal axis, a proximal end and a distal end, the stent having a cylindrical distal section and a split proximal section designed to be flared outward with respect to the cylindrical distal section.

11. The stent of claim 10 where at least some portion of the split proximal section includes a radiopaque material to enhance the radiopacity of the split proximal section.

12. The stent of claim 11 where the radiopaque material is coated onto the exterior surface of some portion of the split proximal section.

13. The stent of claim 11 where the radiopaque material is inserted into one or more holes in the split proximal section.

14. The stent of claim 11 where the radiopaque material includes gold, platinum, tantalum, iridium, palladium or tungsten, or alloys thereof.

15. The stent of claim 13 where the split proximal section further comprises individual spokes, with each spoke having a radiopaque insert.

16. The stent of claim 13 where the split proximal section further comprises individual spokes with every other spoke having a radiopaque insert.

17. The stent of claim 13 where the split proximal section further comprises individual spokes with less than half of the spokes having a radiopaque insert.
